# Europäisches Patentamt
## European Patent Office
### Office européen des brevets

(11) Publication number: **0 180 708**
**B1**

## EUROPEAN PATENT SPECIFICATION

(45) Date of publication of patent specification: **17.05.89**

(51) Int. Cl.⁴: **A 61 M 31/00**

(21) Application number: **85106110.1**

(22) Date of filing: **08.03.83**

(60) Publication number of the earlier application in accordance with Art. 76 EPC: **0 089 548**

(54) Osmotic drug delivery device.

<table>
<tr><td>

(30) Priority: **18.03.82 US 359447**
**18.03.82 US 359324**

(43) Date of publication of application:
**14.05.86 Bulletin 86/20**

(45) Publication of the grant of the patent:
**17.05.89 Bulletin 89/20**

(84) Designated Contracting States:
**AT BE CH DE FR GB IT LI LU NL SE**

(56) References cited:
**EP-A-0 040 457**
**FR-A-2 386 316**
**US-A-4 210 139**

</td><td>

(73) Proprietor: **MERCK & CO. INC.**
**126, East Lincoln Avenue P.O. Box 2000**
**Rahway New Jersey 07065-0900 (US)**

(72) Inventor: **Higuchi, Takeru**
**2811 Schwarz Road**
**Lawrence Kansas 66044 (US)**
Inventor: **Himmelstein, Kenneth J.**
**1640 Hillcrest**
**Lawrence Kansas 66044 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to an osmotic drug delivery device shaped and sized for oral ingestion, implantation, rectal, vaginal or ocular insertion, for delivery of a drug or other beneficial substance. The device comprises a compartment consisting of a chamber. The chamber contains osmotically active agents, adsorption adjuvants, pro-drugs, enzymes, drugs, pesticides or other beneficial substance. The chamber is formed by an external shell of a rigid microporous material and has an overlayer of a semipermeable membrane substantially covering the external shell.

Osmotic drug delivery systems make possible the administration of an active agent at a controlled and continuous rate to achieve a predetermined useful effect in animals including humans.

An osmotic drug delivery device is known from EP—A—0 040 457 which consists of two chambers and in which the chamber-dividing wall is semipermeable or impermeable to fluid agents and solutes. Part of the exterior of the chamber containing the osmotic agent may be of a semipermeable material. The osmotic pressure in the one chamber decreases the volume of the other chamber containing the drug so that it is dispensed through the microporous shell. Because the osmotic pressure is transmitted to the drug-containing chamber through deformation of the chamber-dividing wall the drug delivery rate is influenced by outside agitation.

FR—A—2,386,316 discloses an osmotic drug delivery device in which the drug is dispensed through a minute orifice in the wall of the device. Such a device tends to administer the active agents at a relatively small area of body tissue. Due to the relatively high concentration, local toxic reactions may be caused and the absorption of the drug may be decreased. In addition, the orifice through which the active agent is dispensed can be subject to mechanical plugging.

The invention as claimed in claim 1 solves the problem of how to design an osmotic delivery system whose release rate is independent of outside agitation and which avoids local toxic reactions.

The advantages offered by the invention are mainly that the drug is dispensed at a controlled rate for a prolonged period of time, that the device is designed with a minimum number of parts, that a complete pharmaceutical dosage regimen for a particular time period can be administered, the user of which requires intervention only for initiation and termination of the regimen, that a high concentration of an active agent can be contained therein and that the high concentration of the active agent will not exhibit the tendency to be leached from the device nor have its potency decreased by chemical breakdowns. The device may contain a product which can be used as an osmotically effective solute to exhibit an osmotic pressure gradient against an external fluid. The device of the invention releases the drug at a rate which does not vary with time. The device can contain the drugs in various physiochemical forms such as esters, salts and the like that can be heat sterilized by conventional techniques. The release rate can be chosen ranging from very low to very high by using polymeric wall forming materials in combination with the active agent or mixture of an active agent and other osmotically effective compound. The device can be made from erodible or biodegradable materials that erode or degrade after the device has released the active agent.

The parent patent EP—B—0 089 548 also relates to an osmotic drug delivery device.

Preferred embodiments of the invention are subject matter of the subclaims. Several ways of carrying out the invention are described in detail below with reference to the drawing, in which:—

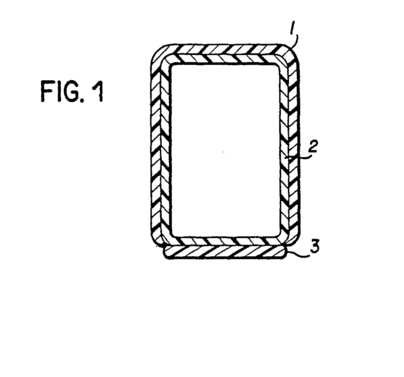

Figure 1 is a cross-section of the osmotic drug delivery device.

The osmotic drug delivery device comprises a compartment consisting of one chamber, the chamber containing osmotically active agents, adsorption adjuvants, drugs, and pro-drugs, the chamber being formed by an external shell 2, the external shell 2 being of a rigid microporous material; one overlayer of a semipermeable membrane 1, the overlayer substantially covering the external shell 2 of the chamber leaving exposed a microporous drug-emitting surface; and optionally including a quick release loading dose of drug external to and covering the microporous drug-emitting surface designed for spontaneous removal following establishment of desired flow characteristics.

The osmotically active agent may or may not be a pharmacologically active agent or pro-drug, especially a relatively soluble pro-drug form of a relatively insoluble drug. The shell 2 of that chamber is designed to be relatively inflexible but semipermeable to water. The semipermeability to water and suitable rigidity can be obtained, for example, by coating a microporous, rigid shell 2 of a relatively hard, preferably biodegradable polymer, with an appropriate semipermeable membrane 1. The pores of the shell 2 of the chamber may or may not be filled with the osmotically active agent.

The single chamber houses both the drug or other beneficial substance and appropriate osmotic agents. Water is imbided through all the walls of the entire device; osmotic pressure is increased forcing the resulting drug solution from the device through the exposed microporous shell 2.

In embodiments of the novel device wherein an appreciable portion of the drug-emitting surface must be exposed, it is convenient to employ a cap 3 that will spontaneously be removed when proper flow characteristics are achieved. Spontaneous removal can be the result of achieving some predetermined internal osmotic pressure in which case the cap 3 is composed of a slowly bioerodible material. On the other hand, if the cap can be readily expelled from the body it can be made of quite impervious materials. In another mode of operation, the cap is composed of a bioerodible material having dispersed therein a loading dose of the drug or other beneficial substance.

In its operation, the device is introduced to the appropriate site such as the rectum, muscle, gastrointestinal tract, vagina, or cul de sac. Because of the substantial area of the semipermeable membrane 1 exposed by the device, water is slowly drawn into the chamber from the adjacent tissue by osmotic action, at a rate controlled by the water permeability of the semipermeable membrane 1. The aqueous solution which is produced in the chamber at a more or less constant rate by this process flows through and carries with it the drug substance stored in the chamber through the microporous shell 2. The overall rate is determined by the total osmotic volume flow rate generated across the semipermeable membrane 1 and the amount of the drug substance transported to the surface of the device per unit volume of the osmotically-produced aqueous fluid.

Another embodiment of the present invention includes a quick-releasing loading dose of the drug substance external to the drug reservoir on the surface of the microporous shell 2 which would eventually serve as the drug-emitting surface. This is a desirable feature since the pumping action will not take place immediately on insertion of the device.

The substance forming a large part of the outer surface of the device is semipermeable, for example a material that is permeable to an external fluid such as water and the like while essentially impermeable to a selected product or other compounds in the device. This material can be non-erodible or bioerodible after a predetermined period of time and in each instance it is semipermeable to solvent but not to solute and is suitable for construction of the outer layer of the device. Typical materials for forming the wall include membranes known to the art as osmosis and reverse osmosis membranes such as commercially available unplasticized cellulose acetate, plasticized cellulose acetate, reinforced cellulose acetate, cellulose nitrate with 11 percent nitrogen, cellulose diacetate, cellulose triacetate, agar acetate, amylose triacetate, beta glucan acetate, beta glucan triacetate, cellulose acetate, acetaldehyde dimethyl acetate, cellulose acetate ethyl carbamate, cellulose acetate phthalate, cellulose acetate methyl carbamate, cellulose acetate succinate, cellulose acetate dimethaminoacetate, cellulose acetate ethyl carbonate, cellulose acetate chloroacetate, cellulose acetate ethyl oxalate, cellulose acetate methyl sulfonate, cellulose acetate butyl sulfonate, cellulose acetate proprionate, cellulose acetate p-toluene sulfonate, triacetate or locust gum bean, cellulose acetate with acetylated hydroxyethyl cellulose, hydroxylated ethylene-vinylacetate, cellulose acetate butyrate having a viscosity of from about 10 seconds to about 50 seconds, cellulose acetate butyrate containing about 17 percent of combined butyryl and about 29.5 per cent acetyl, permselective, aromatic nitrogen-containing polymeric membranes that exhibit water permeability and essentially no solute passage, osmosis membranes made from polymeric epoxides, osmosis membranes made from copolymers of an alkylene oxide and alkyl glycidyl ether, semi-permeable polyurethanes, semi-permeable polyglycolic or polylactic acid and derivatives thereof, thin film membranes as disclosed by Loeb and Sourirajan in US—A—3,133,132, the membranes of ionically associated polyelectrolytes, the polymers formed by the coprecipitation of a polycation and a polyanion as described in US—A-3,276,586; 3,541,005; 3,541,006; 3,546,142; 3,173,876; derivatives of polystyrene such as poly(-sodium styrenesulfonate) and poly(vinylbenzyltrimethyl-ammonium chloride), and the like. Generally, membranes, having a fluid permeability of 0.01 to 10 cc/cm²/hour or day/or higher at atmosphere pressure against a saturated product solution or saturated solute solution to a changing concentration at the temperature of use while simultaneously possessing a high degree of impermeability to the product or solute are useful.

The preferred materials are the cellulose acetates, especially cellulose triacetate.

The microporous material from which the rigid shell 2 is composed can be described as having a sponge-like appearance that provides a supporting structure for microscopic-sized interconnected pores or voids. The materials can be isotropic wherein the structure is homogenous throughout a cross-sectional area, or they can be anisotropic wherein the structure is non-homogenous throughout a cross-sectional area. The pores can be continuous pores that have an opening on both faces of microporous material, pores interconnected through tortuous paths of regular and irregular shapes including curved, curved-linear, randomly oriented continuous pores, and other porous paths discernible by microscopic examination. Generally microporous materials are defined by the pore size, the number of pores, the tortuosity of the microporous path and the porosity which relates to the size and the number of pores. The pores size of microporous material is easily ascertained by measuring the observed pore diameter at the surface of the material under the electron microscope. Generally, materials possessing from 5 to 95% pores and having a pore size of from 1 nm (10 angstroms) to about 100 μm can be used for making the device. The pore size and other parameters characterizing the microporous structure also can be obtained from flow measurements as discussed in US—A—3,977,404.

Microporous materials are commercially available materials and can be made by art known methods. The materials can be made by etched nuclear tracking, by cooling a solution of flowable polymer below the freezing point whereby solvent evaporates from the solution in the form of crystals dispersed in the polymer and then curing the polymer followed by removing the solvent crystals, by cold or hot stretching at low or high temperatures until pores are formed, by leaching from a polymer a soluble component by an appropriate solvent, by ion exchange reaction, and by polyelectrolyte processes. Processes for preparing microporous materials are described in Synthetic Polymer Membranes, by R. E. Kesting, Chapters 4 and 5, 1971, published by McGraw Hill, Inc.; Chemical Reviews, Ultrafiltration. Vol. 18, pages 373 to 455, 1934; Polymer Eng. and Sci., Vol. 11, No. 4, pages 284 to 288, 1971; J. Appl. Poly. Sci., Vol. 15, pages 811 to 829, 1971; and in US—A—3,565,259, 3,615,024; 3,751,536; 3,801,692, 3,852,224; and 3,849,528.

Microporous materials useful for making the devices include microporous polycarbonates comprised of linear polyesters of carbonic acid in which carbonate groups recur in the polymer chain, microporous materials prepared by the phosgenation of a dihydroxyl aromatic such as bisphenol A, poly(vinylchloride), microporous polyamides such as polyhexamethylene adipamide, microporous modacrylic copolymers including those formed for poly(vinylchloride) 60% and acrylonitrile, styrene-acrylic and its copolymers, porous polysulfones characterized by diphenylene sulfone groups in a linear chain thereof, halogenated poly(vinylidene), poly-chloroethers, acetal polymers, polyesters prepared by esterification of a dicarboxylic acid or anhydride with an alkylene polyol, poly(alkylenesulfides), phenolic polyesters, asymmetric porous polymers, cross-linked olefin polymers, hydrophobic or hydrophilic microporous homopolymers, copolymers or interpolymers having a reduced bulk density, and materials described in US—A—3,595,752; 3,643,178; 3,654,066; 3,709,774; 3,718,532; 3,803,061; 3,852,224; 3,853,601; and 3,852,388, in GB—A—1,126,849, and in Chem. Abst., Vol. 71, 4274f, 22572f, 22573f, 1969.

Additional microporous materials include, polyolefins, poly(urethanes), cross-linked, chain-extended poly(urethanes), microporous poly(urethanes) in US—A—3,524,753, poly(imides), poly(benzimidazoles), collodion (cellulose nitrate with 11% nitrogen), regenerated proteins, semisolid cross-linked poly(vinylpyrrolidone), microporous materials prepared by diffusion of multivalent cations into polyelectrolyte sols as in US—A—3,565,259, anisotropic permeable microporous materials of ionically associated polyelectrolytes, porous polymers formed by the coprecipitation of a polycation and a polyanion as described in US—A—3,276,589, 3,541,005, 3,541,006, and 3,546,142, derivatives of poly(styrene) such as poly(sodium styrenesulfonate) and poly(vinyl benzyltrimethylammonium choride), the microporous materials disclosed in US—A—3,615,024, 3,646,178 and 3,852,224. Other microporous materials include those that slowly erode over time, or erode after the device has released the agent; such as, cross-linked gelatin, cross-linked poly(lactide), cross-linked poly(vinyl alcohol) and poly(glycolide).

The preferred microporous materials are fabricated from cellulosics described earlier, preferably the celluose triacetate.

Representatives of compositions of matter that can be released from the device and can function as a solute are without limitation those compositions soluble in aqueous type fluids such as tear fluid, tissue juices, water; organic solvents and the like. The expression "composition of matter" as used in this disclosure is meant to include the terms product, active agent, beneficial agent and the like, and these terms are deemed as functionally equivalent for the present invention. These compositions are osmotically effective as solutes since they can generate a solvent concentration gradient between the exterior medium and the medium inside the device. These compositions include organic and inorganic compounds such as ephedrine hydrochloride, ephedrine sulfate, hydroxyamphetamine, isoproterenol hydrochloride, carbachol, pilocarpine hydrochloride, pilocarpine nitrate, demecarium bromide, ecothiophate iodide, physostigmine salicylate, timolol maleate, homatropine hydrochloride, homatropine methylbromide, methscopolamine nitrate, alverine citrate, chlorphenoxamine hydrochloride, calcium pantothenate and the like. Additional compositions that can be administered are those that produce a physiologically or pharmacalogically useful effect at a point in near relation to the delivery device, or compositions that will produce a physiological or pharmacological response at a site remote from the point of release from the device include drugs generically known as, without limitation, hypnotics, sedatives, psychic energizers, tranquilizers, anti-convulsants, muscle relaxants, analgesics, anti-inflammatories, anesthetics, anti-spasmodics, anti-ulcer agents, anti-microbials, hormonal agents, cardiovascular agents, diuretics, neoplastic agents, and the like.

The composition, drug of the like can also be in various forms, such as uncharged molecules, components of molecular complexes, pharmacologically acceptable salts such as hydrochloride, hydrobromide, sulfate, phosphate, nitrate, borate, acetate, maleate, tartrate, salicylate and the like. For acidic drugs, salts of metals, amines, or organic cations, for example quarternary ammonium can be employed. Furthermore, simple derivatives of the drug such as esters, ethers, amides, and the like which have good solubility characteristics are suitable for the purpose of the invention. Also, a product or drug that is water insoluble can be used in a form that is a water soluble derivate thereof to effectively serve as a solute, and on its release from the device is converted by enzymes, hydrolyzed by body pH, or other metabolic processes to the original form or to a biologically active form. Additionally, the drug formulation can have various art known forms such as solution, dispersion, paste, cream, particle, granule, tablet, emulsions, suspensions, powders and the like.

Various osmotically effective solutes including organic and inorganic compounds are advantageously used when it is desired to release a composition, product, drug or the like having limited solubility from the device. The term "limited solubility" as used herein means that the compound has a solubility of less than about 1% by weight in the external fluid, that is, the ratio of the weight of the compound in solution to the weight of the water of that solution is less than 1 percent. The term includes low, slightly and moderate solubility of the composition in the field. The osmotically effective compounds or solutes confined in the device are a substantial motive force of the device and they exhibit an osmotic pressure gradient against an external fluid across the membrane while the membrane is substantially impermeable to the passage of the osmotically effective solute to prevent loss thereof through the membrane. The solutes are conveniently used by dispensing or homogeneously or heterogeneously mixing a solute or a mixture of solutes with the composition, active agent, product or the like either before they are charged into the compartment or by self mixing after charging a solute and composition into the compartment. In operation, these solutes osmotically attract fluid into the device to produce a solution of the solute which is released from the device concomitantly transporting therewith undissolved and dissolved composition, product, drug or the like. Various osmotically effective solutes include compounds such as magnesium sulfate, magnesium chloride, sodium chloride, lithium chloride, potassium sulfate, sodium carbonate, sodium sulfite, lithium sulfate, calcium bicarbonate, sodium sulfate, calcium sulfate, potassium acid phosphate, calcium lactate, magnesium succinate, tartaric acid, soluble carbohydrates such as raffinose, glucose, mixtures thereof and the like. The solid solute, present initially in excess, can be in any suitable physical form such as particles, crystals, pellets, tablets, strips, film, granules and the like.

The preferred osmotic agents are sodium chloride, sodium carbonate, and calcium bicarbonate.

Additionally, the composition and composition solute can be used in a mixed form by mixing the composition or product with a binder. The product in powdered, granular, piece and the like form, is homogeneously or heterogeneously dispersed in the binder which binder is water soluble or water insoluble but will release the product on contact with water. Typical water soluble binders include polyethylene glycol, gelatin, agar, carboxycellulose, ethylmethylcellulose, polyvinyl alcohol, polyvinylpyrrolidone, water soluble starch derivatives and the like. Typical water insoluble binders that can comprise about 1 to 50 percent of the composition include cellulose acetate, polyurethane, epoxides, and other insoluble binders that permit the free movement of water into the pores of the structure to transport the product from the binder.

The amount of composition present in the device, whether soluble, a derivatized soluble form thereof, is generally non-limited and it is an amount larger than or equal to the amount of the composition that is necessary to osmotically operate the device and on its release from the device is effective for bringing about the product's desired effect. Since the invention contemplates a variety of devices of various sizes and shapes, for a variety of uses, there is no critical upper limit on the amount of product incorporated in the device. The lower limit will depend on osmotic activity, the span of the release of the product and the activity of the product. Generally, the device will contain about 0.01 percent to 90 percent or higher of a product or a mixture of product and solute based on the weight of the product or product solute to the volume of the device, and the like. Typically, the device can be of such size and shape to release 0.01 cc to 5 cc or higher of product contained in the fluid per hour, day or longer, such as 1 cc to 10 cc of product solution for 1 to 10 days, and the like.

The technology relating to the fabrication of the novel device of this invention is well known in the pharmaceutical art.

## Example

A mixture of 100 mg sodium indomethacin-trihydrate and potassium bicarbonate is compressed into a tablet with a surface area of 1.2 cm². The tablet is coated with a microporous layer of cellulose triacetate of 25% porosity and a thickness of .025 cm. The average size of the pores is 20µ radius. The tablet is then overcoated with a semipermeable membrane of cellulose triacetate which will produce a volumetric flow rate of about .07 ml/hr. The overcoating leaves an uncoated microporous layer of 20% of the table area.

## Claims

1. An osmotic delivery device shaped and sized for oral ingestion, implantation, rectal, vaginal or ocular insertion, for delivery of a drug or other beneficial substance comprising a compartment consisting of a chamber containing an osmotically active agent and a drug or other beneficial substance, the chamber being formed by an external shell (2) of a rigid microporous material and having an overlayer of a semipermeable membrane (1), the overlayer substantially covering the external shell (2), characterized in that the overlayer of a semipermeable membrane leaves exposed a microporous drug-emitting surface.

2. The osmotic delivery device of claim 1, wherein a quick release loading dose is external to and covers the microporous drug-emitting surface.

3. The osmotic delivery device of claim 1 or 2, wherein a cap (3) covers the drug-emitting surface designed for spontaneous removal following establishment of desired flow characteristics.

4. The osmotic delivery device of any one of claims 1 to 3 wherein the microporous material of the external shell (2) and the semipermeable membrane (1) of the overlayer are fabricated from a cellulose acetate.

## Patentansprüche

1. Eine durch Osmose wirksame Abgabevorrichtung, die in Form und Größe für orale Einnahme, Implantation, rektales, vaginales oder okulares Einlegen bestimmt ist, für die Abgabe eines Arzneimittels oder einer anderen nützlichen Substanz, umfassend eine aus einer Kammer bestehende Abteilung, die ein osmotisch wirksames Mittel und ein Arzneimittel oder eine andere nützliche Substanz enthält, wobei die Kammer durch ein Außengehäuse (2) aus einem steifen, mikroporösen Material gebildet ist und eine Auflage aus einer semipermeablen Membran (1) aufweist, wobei die Auflage das Außengehäuse (2) im wesentlichen bedeckt, dadurch gekennzeichnet, daß die Auflage aus einer semipermeablen Membran eine mikroporöse, Arzneimittel emittierende Oberfläche exponiert läßt.

2. Die durch Osmose wirksame Abgabevorrichtung des Anspruchs 1, wobei eine rasch freisetzbare Belastungsdosis außerhalb der mikroporösen, Arzneimittel emittierenden Oberfläche vorliegt und diese bedeckt.

3. Die durch Osmose wirksame Abgabevorrichtung des Anspruchs 1 oder 2, wobei eine Kappe (3) die Arzneimittel emittierende Oberfläche bedeckt und zur spontanen Entfernung nach Einstellung der gewünschten Fließkennmerkamale vorgeshen ist.

4. Die durch Osmose wirksame Abgabevorrichtung eines der Ansprüche 1 bis 3, wobei das mikroporöse Material des Außengehäuses (2) und die semipermeable Membran (1) der Auflage aus einem Celluloseacetat hergestellt sind.

## Revendications

1. Dispositif osmotique de libération ayant une forme et une taille convenant à l'ingestion orale, à l'implantation et à l'insertion rectale, vaginale ou oculaire pour la libération d'un médicament ou d'une autre substance bénéfique, comprenant un compartiment constitué d'une chambre contenant un agent à activité osmotique et un médicament ou une autre substance bénéfique, la chambre étant formée d'une enveloppe extérieure (2) faite d'une matière microporeuse rigide et ayant une couche de recouvrement faite d'une membrane semi-perméable (1), la couche de recouvrement recouvrant essentiellement l'enveloppe extérieure (2), caractérisé en ce que la couche de recouvrement d'une membrane semi-perméable laisse apparaître une surface microporeuse émettrice de médicament.

2. Dispositif osmotique de libération de la revendication 1, dans lequel une dose d'attaque à libération rapid est située à l'extérieur de la surface microporeuse émettrice de médicament qu'elle recouvre.

3. Dispositif osmotique de libération de la revendication 1 ou 2, dans lequel une coiffe (3) recouvre la surface émettrice de médicament et est conçue pour être spontanément éliminée après l'établissement des caractéristiques désirées d'écoulement.

4. Dispositif osmotique de libération selon l'une quelconque des revendications 1 à 3, dans lequel la matière microporeuse de l'enveloppe externe (2) et la membrane semi-perméable (1) de la couche de recouvrement sont faites d'un acétate de cellulose.

EP 0 180 708 B1

FIG. 1